(19) Europäisches Patentamt
European Patent Office
Office européen des brevets

(11) **EP 3 524 971 A1**

(12) **EUROPEAN PATENT APPLICATION**

(43) Date of publication:
**14.08.2019 Bulletin 2019/33**

(21) Application number: **19156051.5**

(22) Date of filing: **07.02.2019**

(51) Int Cl.:
*G01N 27/414* [(2006.01)]     *G01N 33/483* [(2006.01)]
*C12Q 1/48* [(2006.01)]     *C12Q 1/6825* [(2018.01)]
*C12Q 1/6834* [(2018.01)]

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.02.2018 IT 201800002511**

(71) Applicant: **Università Degli Studi di Cagliari 09124 Cagliari (IT)**

(72) Inventors:
• **BARBARO, Massimo**
  **09131 Cagliari (IT)**
• **LAI, Stefano**
  **09134 Cagliari (IT)**
• **NAPOLI, Corrado**
  **09045 Quartu Sant'Elena (IT)**

(74) Representative: **Baroni, Matteo et al Metroconsult S.r.l. Foro Buonaparte, 51 20121 Milano (IT)**

(54) **METHOD FOR MEASURING TELOMERASE ACTIVITY**

(57) Method for measuring telomerase activity, comprising: providing a field-effect transistor equipped with a control gate and a floating gate, wherein said floating gate has at least one free portion exposed to the outside environment; executing a functionalization step, wherein one or more DNA probes are immobilized on said free portion; executing an interaction step, wherein said one or more DNA probes are made to interact with an analyte associated with telomerase, the activity of which needs to be measured; supplying power and polarization to said field-effect transistor; executing a detection step, wherein an electric parameter associated with said field-effect transistor and representative of the activity of said telomerase is detected.

FIG. 1

Printed by Jouve, 75001 PARIS (FR)

## Description

### Field of application of the invention

The present invention relates to a method for measuring telomerase activity.

### State of the art

Programmed cell death, referred to as apoptosis, is a self-regulated biological process that keeps constant the number of cells in an organism. Under-regulation thereof may be a symptom of several disorders in adult individuals. Such disorders may range from neurodegenerative diseases to ischemic damage, autoimmune diseases and the majority of tumours.

Recent studies have demonstrated that there is a correlation between the limit of possible cell divisions, known in the art as *Hayflick limit*, and cellular senescence. The cell ageing phenomenon is, in turn, dependent on the shortening of the ends of DNA molecules, called *telomeres.* Telomeres are sequences of nucleotides, the main function of which is to protect and give stability to DNA molecules, the progressive shortening of which occurs because of the inability of known DNA polymerases to completely replicate the distal part of one of the two chains forming the double helix, i.e. the slow filament. When the telomeres become too short and reach a critical length, the cells die.

The ribonucleoprotein complex of telomerase is a particular enzyme capable of catalyzing the synthesis of telomeres, thus stabilizing their length [1]. The direct consequence of this elongation process is the possibility for the cells in which it is active to proliferate in an uncontrolled manner. It has been demonstrated that telomerase is not active in most somatic cells, except for embryonic tissues, stem cells and reproductive organs. Recent studies has shown that, conversely, the protein is present and strongly active in most (80% - 90%) human tumours [2]. For this reason, telomerase is a biomarker that can be used for identifying neoplastic transformations and for early cancer diagnosis.

The methods currently in use in test laboratories are mostly based on modifications of the TRAP protocol (*Telomerase Repeat Amplification Protocol*) [1] [3], which allows the execution of semi-quantitative and quantitative analyses through the use of particular fluorescence-marked deoxynucleotide triphosphates (dNTP).

This protocol can be divided into three main phases: elongation of the telomerase template, amplification of the product thus obtained, and, finally, quantification thereof. The intermediate amplification phase is necessary to obtain good sensitivity with quantification methods [4]. The amplification method, based on PCR (*Polymerase Chain Reaction*), is however complex and costly, and requires long preparation times.

In the current state of the art, the only alternative to TRAP-based methods is offered by nanotechnologies.

Several types of approach have been proposed, using different techniques and numerous transduction types, for the purpose of measuring telomerase activity [5]. Most of them are indirect measurement methods. The methods of analysis based on immunological assays do not require the use of fluorescent markers, but still require the use of expensive reagents; the methods based on *Molecular Beacons* and nanoparticles, on the contrary, require fluorescent probes and long production times. The methods that provide a direct measurement are essentially based on field-effect electronic devices (*Field Effect Transistors,* FET). It is known, in fact, that it is possible to use such devices to monitor different types of biochemical reactions by analyzing small quantities of sample, without the latter having to be amplified by means of techniques such as, for example, PCR. In this case, the detection mechanism is substantially related to field-effect modulation. Patolsky *et al.* have proposed and patented a device [6], [7] for detecting cancer markers, which is based on silicon *nanowires* functionalized with probes of oligonucleotides replicating the telomeric sequence. The electric charge of the enzyme and of the nucleotides can be transduced by measuring the conductance of the nanotube. In fact, when telomerase binds to the probes immobilized on the surface, the conductance value varies in accordance with a known law, which allows for direct detection of the reaction that has occurred.

The most limiting factor of this approach is the Debye length. This length is the distance within which it is possible to identify a charge in a solution before the electrostatic shield effect generated by the ionic species in solution makes it impossible to detect the electrostatic field generated by a charged molecule in solution, thus limiting, *de facto*, the possibility of detecting charged molecules by means of devices based on field-effect modulation. Moreover, since biological phenomena occur in solutions having high ionic concentrations, this parameter is commonly indicated as a limiting factor for the detection of charged molecules in solution [8].

The above examples have shown that the techniques currently available for telomerase activity detection suffer from several drawbacks, which depend on the transduction method employed, and which make automatization impossible and prevent the development of a low-cost testing product. Field-effect-based electronic devices represent an interesting alternative to the biochemical procedures currently available on the market, but the limits related to the Debye length are greatly hindering their development.

### Summary of the invention

It is therefore the object of the present invention to provide a method for measuring telomerase activity, which can overcome the limits suffered by prior-art techniques.

In particular, it is the object of the invention to provide a simple, fast and automatable method that also allows evaluating, optionally in real time, telomere elongation.

Brief description of the drawings

These and other objects are substantially achieved through the method for measuring telomerase activity as described in the appended claims.

Further features and advantages will become more apparent in the light of the following detailed description of some preferred and non-limiting embodiments of the invention. This description will refer to the annexed drawings, which are also provided merely as explanatory and non-limiting examples, wherein:

Figure 1 schematically shows the principle of operation of a field-effect transistor, on which the invention is based, by means of an example of implementation in organic electronics;
Figure 2 shows one possible electric characterization of the transistor of Figure 1, for extracting the parameters of interest for the measurement;
Figures 3a, 3b schematically show possible embodiments of field-effect transistors that may be used within the scope of the present invention;
Figures 4-8 show diagrams representing quantities involved in the method according to the present invention.

Detailed description

The method according to the invention makes it possible to measure telomerase activity. Such measurement can be conducted without requiring template amplification by PCR. It is possible to evaluate the product of a biochemical assay (TRAP) either after the primer elongation phase or in real time, during the telomere elongation phase.

Preferably, the method envisages a measurement of the variation occurring in the threshold voltage of a field-effect device following immobilization of DNA chains on the surface of the sensing area, which will be further described hereinafter. In fact, DNA suspended in a buffer solution has a negative net charge due to the phosphate group that is present in the backbone of the molecule. Because the charge of DNA chains depends in a linear manner on the number of nucleotides that make up the chain, it is possible to exploit the principle of operation of the sensor to make a device for direct measurement of telomerase activity.

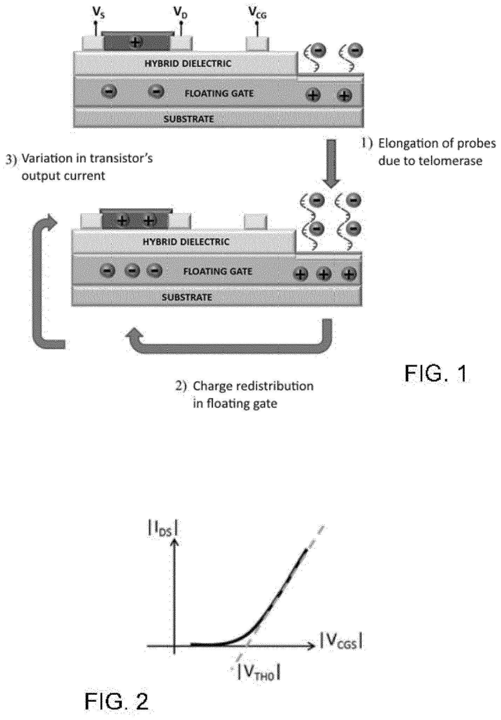

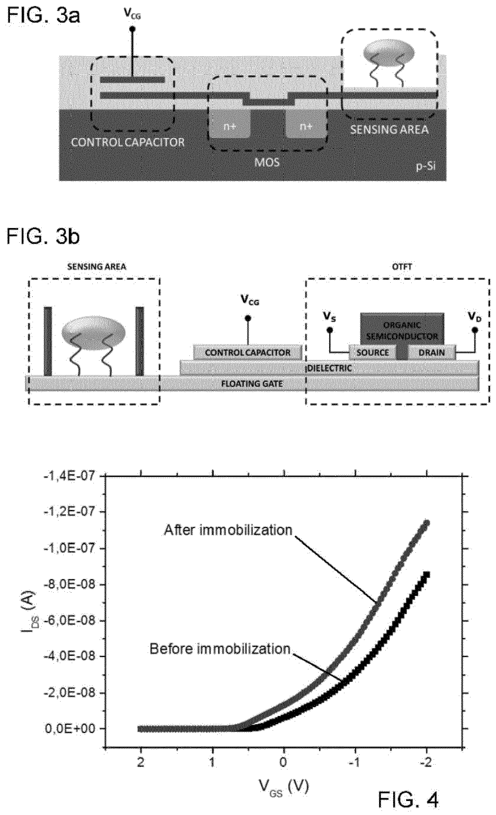

More in detail, the method envisages the use of a field-effect transistor equipped with a floating gate and a control gate (referred to as $V_{CG}$ in Fig. 1 and Figs. 3a and 3b) capacitively coupled to said floating gate. The transistor component is based on a MOS structure, and is generally referred to as FGMOS.

In one embodiment, the transistor may be implemented with CMOS technology. By way of example, the transistor may be made in accordance with the teachings of United States patent US 7,535,232 B2 [10], incorporated herein by reference. A transistor of this kind has been described by *Barbaro et al.* also in another publication [9].

In one embodiment, the transistor may be implemented with organic semiconductor technology, e.g. in accordance with international patent application WO 2016/124714 A1, incorporated herein by reference.

The floating gate of the transistor has at least one free portion exposed to the outside environment. The free portion may also be referred to as "sensing area".

As will become more apparent hereinafter, the free portion is used for taking the measurements of interest.

It should be noted, in particular, that the transistor in question is completely formed and is equipped with a floating gate; this gate electrode is preferably elongated, so that it can be exposed to the outside environment. The current flowing in the channel of this device is not modulated by the effect of the double layer directly formed on the channel, but by the voltage drop across the floating-gate electrode and the substrate. This voltage drop depends, through the field effect, on the quantity of electric charge immobilized on the exposed surface. The control electrode, capacitively coupled to the floating gate, makes it possible to set the working point of the device correctly, bringing the transistor within its optimal working range and letting the charge variations that occur on the floating gate cause a simple current variation relative to the polarization point. In this way, compared to other solutions based on a FET structure, it is possible to increase the limit of detection (LOD) of the device, the activation of which will not depend on the presence of a charge on the floating gate, but on the voltage directly applied to the control gate. Furthermore, thanks to the presence of the control gate, it is possible to overcome the limits imposed by the Debye length, which are characteristic of all other FET-based devices known in the art. By applying a suitable voltage to the control gate, in fact, it is possible to increase the Debye length through two mechanisms: the electrostatic repulsion of free counter-charges (counter-ions), which shield the effect of the immobilized charge, and the bending of the immobilized molecules towards the electrode, thus approaching the sensing surface.

Figures 3a, 3b schematically show the structure that the MOSFET may have, depending on the technology employed for making it. In particular, Figure 3a shows a MOSFET made by means of a standard CMOS process (CMFET), whereas Figure 3b shows one possible bottom-gate, bottom-contact configuration for implementation with an organic semiconductor (OCMFET).

In one embodiment, the method according to the invention envisages the execution of the following steps:

a. providing a field-effect transistor equipped with a floating gate and a control gate, wherein said floating gate has at least one free portion exposed to the outside environment;

b. executing a functionalization step, wherein one or more DNA probes are immobilized on said free portion;

c. providing an analyte comprising telomerase, the activity of which needs to be measured;

d. executing an interaction step, wherein said one or more DNA probes are made to interact with said analyte;

e. supplying power to said field-effect transistor;

f. applying a potential to the control gate;

g. executing a detection step, wherein an electric parameter associated with said field-effect transistor and representative of the activity of said telomerase is detected.

For simplicity, this embodiment will be referred to as "real-time measurement".

In this case, the detection step is carried out during the interaction step. In other words, the detection step and the interaction step are at least partially superimposed.

This means that the activity of the enzyme is measured while the latter is acting upon the DNA probe(s), thus causing the elongation of the respective telomeres.

To this end, before the analyte is made to interact with said one or more DNA probes, said one or more probes are immobilized on the free portion of the floating gate.

The molecule portion directly involved in the catalysis process and in the formation of bonds with the telomeres, also referred to as enzyme site (or active center), is complementary to the TTAGGG sequence and binds to the 3' end of the DNA molecule. The structure and properties of the active center allow recognition of and binding to the substrate (telomere) that is also subject to analysis. For this reason, the probes should be immobilized on the golden surface at the 5' end, and at the opposite end they should contain a telomeric sequence. Therefore, the DNA sequence will preferably be modified with a functional group [F] at its 5' end. In order to avoid self-hybridization and formation of secondary structures typical of guanine-rich nucleic acid sequences, it may be necessary to use a spacer sequence. One example of a sequence that may be used as a probe for this type of measurement is: $[F]$-5'-$(SPACER)_n(TTAGGG)_{2m+1}$-3'.

Preferably, the method envisages a step of preparing the analyte, wherein the latter, prior to interacting with said one or more DNA probes, is dissolved into a buffer solution. Preferably, the cellular extract is prepared by using per se known techniques and methods, e.g. as required when using commercial kits. By way of example, the enzyme is diluted into a buffer solution containing: a mixture of dNTPs, the molar concentration of which depends on the quantity of enzymic extract used;

2-20 mM $MgCl_2$;
1 mM EGTA;
63-630 mM KCl;
1-10 mM EGTA;
0.5-0.005% Tween 20;
20-200 mM Tris-HCl, pH 8.3.

For real-time measurement, the electric parameter to be monitored is preferably indicative of an output current of said field-effect transistor.

More in detail, a given quantity (e.g. 2-6 $\mu$L) of previously prepared and diluted extract must be added into the incubation chamber. By measuring the output current of the transistor, it is possible to evaluate its action in real time.

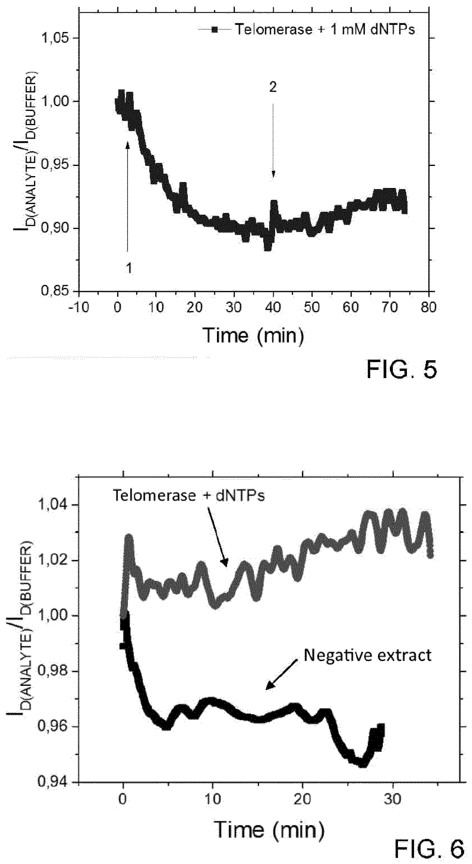

Figure 5 shows an example of real-time measurement of the activity of the enzyme. For the purpose of demonstrating its principle of operation, the different components of the enzyme have been added at different times. After the addition in solution of an active extract of telomerase (1), since the latter has a positive net charge when dissolved into a solution having a pH lower than its isoelectric point (~ 10), the output current of the device decreases. Following the introduction of dNTPs in solution (2), the telomerase can catalyze the synthesis of new telomeric units, thereby elongating the probes immobilized on the sensing area. The increased negative net charge immobilized on the sensing area, due to elongation of the probes, results in an increased output current of the device.

Figure 6 shows an example of a real-time measurement compared with a reference negative extract. In particular, Figure 6 shows the response of the device to a cellular extract containing active telomerase (upper curve) and to a control extract in which the action of the enzyme was inhibited by thermally denaturating (90 °C, 10 minutes) the telomerase (lower curve).

In one embodiment, the method according to the invention comprises the following steps:

a. providing a field-effect transistor equipped with a floating gate, wherein said floating gate has at least one free portion exposed to the outside environment, and equipped with a control gate capacitively coupled to the floating gate;

b. executing a functionalization step, wherein one or more DNA probes are immobilized on said free portion;

c. providing an analyte, causing one or more telomeric sequences to interact with telomerase, the activity of which needs to be measured;

d. executing an interaction step, wherein said one or more DNA probes are made to interact with said analyte;

e. supplying power to said field-effect transistor;

f. supplying a polarization voltage to the control gate;

g. executing a detection step, wherein an electric parameter associated with said field-effect transistor and representative of an activity of said telomerase is detected.

This type of measurement is identified as "static measurement".

In the static measurement case, the detection step is preferably carried out after completion of the interaction step.

In this case, the preparation of the analyte is carried out, for example, by using a commercial kit, and the result thereof is then analyzed.

Preferably, in this case the analyte is prepared before the functionalization step. In other words, the enzyme's action upon the telomeric sequences contained in the analyte is promoted first, and afterwards the DNA probes (complementary to such telomeric sequences) are immobilized on the free portion of the floating gate, so that the measurement according to the invention can be taken.

In the static measurement case, said one or more DNA probes are preferably complementary to a telomeric sequence contained in the analyte.

As aforesaid, for this type of measurement one may use the reaction product of a commercial kit. The template used for this type of analysis is a telomeric sequence amplified by the enzyme to be analyzed. For this type of analysis, the invention substantially replaces the classic qPCR-based quantification techniques. Consequently, the functional group [F] will preferably be located in the 3' position and the probe will have a sequence like: 5'-(CCCGAA)$_{n+1}$(SPACER)$_m$-3'-[F].

Figure 4 shows the variation occurring in the threshold voltage before and after the functionalization of a sensor implemented with organic electronics technology, using a p-type semiconductor. The variation in the threshold voltage, due to the negative charges of the DNA molecules, produces an activation of the device, as predicted by the model shown below. The sequence used is of the following type: 5'-(SPACER)$_{12}$(TTAGGG)$_2$-3'.

Preferably, a step of preparing the analyte is carried out, wherein the latter, prior to interacting with the DNA probes, is filtered and dissolved into a buffer solution. Preferably, the pH of said solution is 6 to 8, and in particular substantially equal to about 7. By way of example, the buffer solution for the static measurement may include:

Tris-HCl 10-50 mM;
EDTA 1 mM;
NaCl 10 mM.

Preferably, the electric parameter representative of the transduction mechanism used is indicative of the threshold voltage of the field-effect transistor.

In particular, a given quantity (e.g. 2-6 μL) of the reaction product obtained by using the kit is filtered and then introduced into the incubation chamber of the sensor.

The activity of the enzyme is quantified by comparing the variation occurring in the threshold voltage of the transistor with the variation occurring in the threshold voltage of a set of control devices.

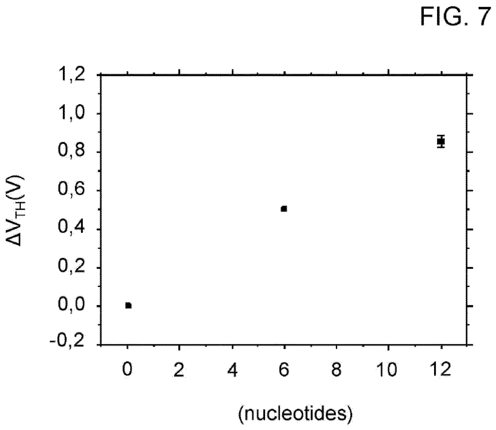

Figure 7 shows an example of a calibration curve that may be used as a reference for the actual measurement of the protein's activity. The threshold voltage variation resulting from the measurement taken on the extract under examination can be compared with the result of a reference calibration curve obtained in a similar way, in order to quantify the template elongation due to the action of telomerase.

In general, the probes can be specifically engineered as a function of the measurement type.

Furthermore, the molecules, in order to be used as probes, are preferably modified with a functional group suitable for binding to the surface of the free portion of the floating gate. The immobilization of the probes on the free portion (sensing area) of the floating gate can be carried out by using different techniques and procedures [12] [13], *per se* known, which will depend on the material used for fabricating the sensing area.

The success of the functionalization step can be verified by means of equation (1), as will be described hereinafter.

As a non-limiting example of a functionalization procedure, one may mention the Self-Assembled Monolayers (SAM) obtained from the chemical bond generated, for example, between a thiol group and a gold surface; on the other hand, more complex alternatives, based on electrochemical procedures, allow the formation of covalent bonds between the molecule and the surface.

As far as the operation of the transistor is concerned, the following should be noted.

Figure 1 schematically shows the principle of operation of the transistor, applied to the specific case of interest: the elongation of DNA molecules immobilized on the surface of the sensing area, caused by telomerase. Because each phosphate group of each nucleotide carries a negative net charge, the variation in the probes' length produces also a variation in the charge immobilized on the surface of the sensing area and a consequent redistribution of the charge within the floating gate. Such redistribution induces a variation in the charge under the area of the transistor's channel and hence, upon polarization of the dielectric, a variation in the current of the device. Barbaro et al. demonstrate that this principle of operation can be modeled as a variation in the threshold voltage of the device according to the following relationship:

$$\Delta V_{TH} = \frac{\Delta Q_S}{C_{SUM}}$$

( 1 )

wherein $Q_S$ represents the charge immobilized on the surface and $C_{SUM}$ represents the sum of the capacities acting upon the device.

Figure 2 shows how, by measuring the variation in the output current of the device as a function of the variation in the voltage applied to the control capacitor, one can extrapolate the threshold voltage of the device.

The telomere shortening problem is connected to the symmetry properties of the DNA molecule. Deoxyribonucleic acid, in fact, is not a symmetrical molecule, and it is possible to define a direction of orientation of the ends of a single filament of nucleic acid. Nomenclature conventions about the sugar rings contained in nucleotides result, as a direct consequence, in the presence of 5' ends and 3' ends. The importance of the existence of this asymmetry becomes apparent when one considers that polymerases that are present in nature are unable to synthesize in the direction 3' → 5', thus leading to the slow filament and incomplete replication problems, which stand at the basis of the existence of the telomere shortening phenomenon and also of the existence of the Hayflick limit. Telomerase is an inverse transcriptase that binds in the 3' position, thereby catalyzing the synthesis of new telomeric units.

Whether one wants to measure the enzyme's action in real time or wants to use the device for analyzing the reaction product of a commercial kit, it is necessary to immobilize DNA molecules on the active surface of the device, which molecules will then act as enzyme probes. This procedure is known as functionalization. It must expressly be pointed out that such probes must be engineered in a specific manner, which will depend on the analysis that needs to be carried out. Furthermore, for the molecules to be used as probes, they must be appropriately modified with a functional group suitable for binding to the surface of the sensing area. Immobilization of the probes on the sensing area can be achieved by using different techniques and procedures[12] [13], *per se* known, which will depend on the material used for fabricating the sensing area. The success of this procedure can be verified by means of equation 1, as previously explained. As a non-limiting example of a functionalization procedure, one may mention the *Self-Assembled Monolayers* (SAM) obtained from the chemical bond generated, for example, between a thiol group and a gold surface; on the other hand, more complex alternatives based on electrochemical procedures allow the formation of covalent bonds between the molecule and the surface.

The elements and features shown in the various preferred embodiments may be combined together without however departing from the protection scope of the present invention. The invention achieves important advantages.

The invention permits overcoming the limitations of sensors based on field-effect modulation [11] and reducing the time necessary for preparing the extract, due to the possibility of analyzing non-amplified samples. In addition, the structure is not subject to problems related to interaction dynamics between semiconductors and oxides and the measurement liquid, which adversely affect most electronic devices used for taking measurements in humid environments. Its implementation is not dependent on the technology employed for fabricating the device, and it is therefore possible to use various types of materials and fabrication processes for implementing the sensor, thus exploiting the advantages offered by different technologies.

The invention allows increasing the limit of detection (LOD) of conventional FET-based devices, due to the fact that the activation of the device is not dependent on the charge to be measured, immobilized on the floating gate, but on the voltage directly applied to the control gate. It is thus possible to detect very small electric charges that would not be able to turn on the transistor, while their modulation effect can be measured once the transistor is already on.

The invention overcomes the limits imposed by the Debye length, which characterize all other FET-based devices known in the art. By applying a suitable voltage to the control gate, in fact, it is possible to increase the Debye length through two mechanisms: the electrostatic repulsion of free counter-charges (counter-ions), which shield the effect of the immobilized charge, and the bending of the immobilized molecules towards the electrode, thus approaching the sensing surface.

In addition to the above, it should be noted that the approach on which the invention is based allows tests to be carried out by using much smaller quantities of reagents (tens of $\mu$L) compared to commercial alternatives, which are essentially TRAP-based.

Furthermore, the method according to the invention can be automated and ensures waiting times reduced to a minimum.

Bibliographic references

[1] N. W. Kim, M. A. Piatyszek, K. R. Prowse, C. Harley, M. D. West, P. L. Ho, G. M. Coviello, W. E. Wright, S. L. Weinrich and J. W. Shay, «Specific association of human telomerase activity with immortal cells and cancer,» Science, vol. 266, no. 5193, pp. 2011-2015, 1994.

[2] E. Blackburn, «Telomerase and Cancer: Kirk A. Landon--AACR prize for basic cancer research lecture.,» Molecular Cancer Research, vol. 3, no. 9, pp. 477-82, 2005.

[3] N. W. Kim, C. B. Harley and S. L. Weinrich, «Telomerase activity assays». US Patent US5629154 A, 13 May 1997.

[4] K. Nam W and W. Fred, «Advances in quantification and characterization of telomerase activity by the telomeric repeat amplification protocol (TRAP),» Nucleic Acids Research, vol. 25, no. 13, p. 2595-2597, 1997.

[5] E. Kulla and E. Katz, «Biosensor Techniques Used for Determination of Telomerase Activity in Cancer Cells,» Sensors, vol. 8, no. 1, pp. 347-369, 2008.

[6] G. Zheng, F. Patolsky, Y. Cui, W. U. Wang and C. M. Lieber, «Multiplexed electrical detection of cancer markers with nanowire sensor arrays.,» Nature biotechnology, vol. 23, no. 10, pp. 1294-1301, 2005.

[7] C. Lieber, F. Patolsky and G. Zheng, «Nanoscale sensors». US Patent US8232584 B2, 31 July 2012.

[8] A. Poghossian, A. Cherstvy, S. Ingebrandt, OffenhäusserA and M. J. Schöning, «Possibilities and limitations of label-free detection of DNA hybridization with field-effect-based devices.,» Sensors and Actuators B: Chemical, Vol. %1 of %2111-112, no. 470-480, 2005.

[9] M. Barbaro, A. Bonfiglio and L. Raffo, «A Charge-Modulated FET for Detection of Biomolecular Processes: Conception, Modeling and Simulation.,» IEEE Transaction on Electron Devices, vol. 53, no. 1, pp. 158-166, 2006.

[10] M. Barbaro, A. Bonfiglio and L. Raffo, «Field-effect device for the detection of small quantities of electric charge, such as those generated in bio-molecular process, bound in the vicinity of the surface». US Patent 7,535,232 B2, 19 May 2009.

[11] S. Lai, M. Barbaro and A. Bonfiglio, «The role of polarization-induced reorientation of DNA strands on organic field-effect transistor-based biosensors sensitivity at high ionic strength,» Applied Physics Letters, vol. 107, no. 10, p. 103301, 2015.

[12] A. Vacca, M. Mascia, S. Rizzardini, S. Palmas, S. Lai, C. Napoli and M. Barbaro, «Functionalization of Polycrystalline Gold Through the Electroreduction of Aryldiazonium Salts in Ionic Liquids,» Chemical Engineering Transactions, vol. 41, pp. 79-84, 2014.

[13] T. M. Heme and M. J. Tarlov, «Characterization of DNA Probes Immobilized on Gold Surfaces,» Journal of the american chemical society, vol. 38, no. 119, p. 8916-8920, 1997.

**Claims**

1. Method for measuring telomerase activity, comprising:

   a. providing a field-effect transistor equipped with a control gate and a floating gate, wherein said floating gate has at least one free portion exposed to the outside environment;
   b. executing a functionalization step, wherein one or more DNA probes are immobilized on said free portion;
   c. providing an analyte comprising telomerase, the activity of which needs to be measured;
   d. executing an interaction step, wherein said one or more DNA probes are made to interact with said analyte;
   e. supplying power to said field-effect transistor;
   f. supplying a polarization voltage to the control gate;
   g. executing a detection step, wherein an electric parameter associated with said field-effect transistor and representative of the activity of said telomerase is detected.

2. Method according to claim 1, wherein said detection step is carried out during said interaction step.

3. Method according to claim 1 or 2, wherein said interaction step is carried out after said functionalization step.

4. Method according to any one of the preceding claims, wherein said one or more DNA probes are complementary to an active site of the telomerase contained in said analyte.

5. Method according to any one of the preceding claims, wherein, during said interaction step, telomeric chains of said one or more DNA probes interact with the telomerase contained in said analyte.

6. Method according to any one of the preceding claims, comprising a step of preparing said analyte, wherein said analyte, prior to interacting with said one or more DNA probes, is dissolved into a buffer solution.

7. Method according to any one of the preceding claims, wherein said electric parameter, representative of the transduction mechanism used, is indicative of an output current of said field-effect transistor.

8. Method for measuring telomerase activity, comprising:

   a. providing a field-effect transistor equipped with a control gate and a floating gate, wherein said floating gate has at least one free portion exposed to the outside environment;

b. executing a functionalization step, wherein one or more DNA probes are immobilized on said free portion;

c. providing an analyte, causing one or more telomeric sequences to interact with telomerase, the activity of which needs to be measured;

d. executing an interaction step, wherein said one or more DNA probes are made to interact with said analyte;

e. supplying power to said field-effect transistor;

f. supplying a polarization voltage to the control gate;

g. executing a detection step, wherein an electric parameter associated with said field-effect transistor and representative of an activity of said telomerase is detected.

9. Method according to claim 8, wherein said detection step is carried out after completion of the interaction step.

10. Method according to claim 8 or 9, wherein said interaction step is carried out before said functionalization step.

11. Method according to any one of claims 8 to 10, wherein said one or more DNA probes are complementary to said one or more telomeric sequences contained in said analyte.

12. Method according to any one of claims 8 to 11, wherein said analyte comprises said one or more telomeric sequences amplified by said telomerase.

13. Method according to any one of claims 8 to 12, wherein, during said interaction step, telomeric chains of said one or more DNA probes interact with said one or more telomeric chains contained in said analyte.

14. Method according to any one of claims 8 to 13, comprising a step of preparing said analyte, wherein said analyte, prior to interacting with said one or more DNA probes, is filtered and dissolved into a buffer solution.

15. Method according to any one of claims 8 to 14, wherein said electric parameter representative of the transduction mechanism used is indicative of a threshold voltage of said field-effect transistor.

16. Method according to any one of the preceding claims, wherein said field-effect transistor is implemented with CMOS technology.

17. Method according to any one of the preceding claims, wherein said field-effect transistor is implemented with organic semiconductor technology - OCMFET.

3) Variation in transistor's output current

1) Elongation of probes due to telomerase

2) Charge redistribution in floating gate

FIG. 1

FIG. 2

## FIG. 3a

## FIG. 3b

FIG. 4

FIG. 5

FIG. 6

FIG. 7

Europäisches
Patentamt

European
Patent Office

Office européen
des brevets

**EUROPEAN SEARCH REPORT**

Application Number

EP 19 15 6051

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ETERY SHARON ET AL: "Optical, Electrical and Surface Plasmon Resonance Methods for Detecting Telomerase Activity", ANALYTICAL CHEMISTRY, vol. 82, no. 20, 15 October 2010 (2010-10-15), pages 8390-8397, XP055516402, US ISSN: 0003-2700, DOI: 10.1021/ac101976t | 1-14 | INV. G01N27/414 G01N33/483 C12Q1/48 C12Q1/6825 C12Q1/6834 |
| Y | * abstract ; Fig 1 ; pg 8391, col 2, para 1 ; pg 8392, col 1, para bridging col 2 ; pg 8392, col 2, para bridging pg 8393 - pg 8393, col 2, l 6 * | 15-17 | |
| X | GENGFENG ZHENG ET AL: "Multiplexed electrical detection of cancer markers with nanowire sensor arrays", NATURE BIOTECHNOLOGY, vol. 23, no. 10, 18 September 2005 (2005-09-18), pages 1294-1301, XP055516542, ISSN: 1087-0156, DOI: 10.1038/nbt1138 | 1-14 | |
| Y | * abstract ; Fig 4 ; pg 1298, col 2, last para , pg 1300, col 1, last full para * | 15-17 | TECHNICAL FIELDS SEARCHED (IPC) G01N C12Q |
| X | US 2006/269927 A1 (LIEBER CHARLES M [US] ET AL) 30 November 2006 (2006-11-30) | 1-14 | |
| Y | * paragraphs [0113], [0189], [0143], [0194]; figures 8A, 8B * | 15-17 | |
| Y | US 7 535 232 B2 (CONSIGLIO NAZIONALE RICERCHE [IT]) 19 May 2009 (2009-05-19) * col 1, l 60 - col 2, l 16 ; col 3, l 43 - 63 ; Fig 7 * | 15-17 | |

-/--

The present search report has been drawn up for all claims

| Place of search | Date of completion of the search | Examiner |
|---|---|---|
| The Hague | 4 June 2019 | Vadot-Van Geldre, E |

page 1 of 2

|  | Europäisches Patentamt European Patent Office Office européen des brevets | **EUROPEAN SEARCH REPORT** | Application Number EP 19 15 6051 |
|---|---|---|---|

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2017/024049 A1 (PACIFIC BIOSCIENCES CALIFORNIA INC [US])<br>9 February 2017 (2017-02-09)<br>* paragraphs [0124], [0231], [0233]; claim 1 * | 1-14<br><br>15-17 | |
| A | S. LAI ET AL: "The role of polarization-induced reorientation of DNA strands on organic field-effect transistor-based biosensors sensitivity at high ionic strength",<br>APPLIED PHYSICS LETTERS,<br>vol. 107, no. 10,<br>7 September 2015 (2015-09-07), page 103301, XP055593844,<br>US<br>ISSN: 0003-6951, DOI: 10.1063/1.4930303<br>* pg 2, col 1, para 3; figures 1, 2a * | 1-17 | |
| | | | **TECHNICAL FIELDS SEARCHED (IPC)** |

The present search report has been drawn up for all claims

| Place of search | Date of completion of the search | Examiner |
|---|---|---|
| The Hague | 4 June 2019 | Vadot-Van Geldre, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 15 6051

This annex lists the patent family members relating to the patent documents cited in the above-mentioned European search report.
The members are as contained in the European Patent Office EDP file on
The European Patent Office is in no way liable for these particulars which are merely given for the purpose of information.

04-06-2019

| Patent document cited in search report | | | Publication date | Patent family member(s) | | Publication date |
|---|---|---|---|---|---|---|
| US 2006269927 | A1 | | 30-11-2006 | NONE | | |
| US 7535232 | B2 | | 19-05-2009 | EP | 1759193 A1 | 07-03-2007 |
| | | | | US | 2007247170 A1 | 25-10-2007 |
| | | | | WO | 2005121765 A1 | 22-12-2005 |
| WO 2017024049 | A1 | | 09-02-2017 | CN | 108139345 A | 08-06-2018 |
| | | | | EP | 3332032 A1 | 13-06-2018 |
| | | | | EP | 3332033 A1 | 13-06-2018 |
| | | | | US | 2017037462 A1 | 09-02-2017 |
| | | | | US | 2017038333 A1 | 09-02-2017 |
| | | | | US | 2019106741 A1 | 11-04-2019 |
| | | | | US | 2019136311 A1 | 09-05-2019 |
| | | | | WO | 2017024017 A1 | 09-02-2017 |
| | | | | WO | 2017024049 A1 | 09-02-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7535232 B2
- WO 2016124714 A1
- US 5629154 A
- US 8232584 B2

### Non-patent literature cited in the description

- **N. W. KIM ; M. A. PIATYSZEK ; K. R. PROWSE ; C. HARLEY ; M. D. WEST ; P. L. HO ; G. M. COVIELLO ; W. E. WRIGHT ; S. L. WEINRICH ; J. W. SHAY.** Specific association of human telomerase activity with immortal cells and cancer. *Science,* 1994, vol. 266 (5193), 2011-2015
- **E. BLACKBURN.** Telomerase and Cancer: Kirk A. Landon--AACR prize for basic cancer research lecture. *Molecular Cancer Research,* 2005, vol. 3 (9), 477-82
- **N. W. KIM ; C. B. HARLEY ; S. L. WEINRICH.** *Telomerase activity assays*
- **K. NAM W ; W. FRED.** Advances in quantification and characterization of telomerase activity by the telomeric repeat amplification protocol (TRAP). *Nucleic Acids Research,* 1997, vol. 25 (13), 2595-2597
- **E. KULLA ; E. KATZ.** Biosensor Techniques Used for Determination of Telomerase Activity in Cancer Cells. *Sensors,* 2008, vol. 8 (1), 347-369
- **G. ZHENG ; F. PATOLSKY ; Y. CUI ; W. U. WANG ; C. M. LIEBER.** Multiplexed electrical detection of cancer markers with nanowire sensor arrays. *Nature biotechnology,* 2005, vol. 23 (10), 1294-1301
- **C. LIEBER ; F. PATOLSKY ; G. ZHENG.** *Nanoscale sensors*
- **A. POGHOSSIAN ; A. CHERSTVY ; S. INGEBRANDT ; OFFENHÄUSSERA ; M. J. SCHÖNING.** Possibilities and limitations of label-free detection of DNA hybridization with field-effect-based devices. *Sensors and Actuators B: Chemical,* 2005, vol. 1, 2111-112
- **M. BARBARO ; A. BONFIGLIO ; L. RAFFO.** A Charge-Modulated FET for Detection of Biomolecular Processes: Conception, Modeling and Simulation. *IEEE Transaction on Electron Devices,* 2006, vol. 53 (1), 158-166
- **M. BARBARO ; A. BONFIGLIO ; L. RAFFO.** *Field-effect device for the detection of small quantities of electric charge, such as those generated in bio-molecular process, bound in the vicinity of the surface*
- **S. LAI ; M. BARBARO ; A. BONFIGLIO.** The role of polarization-induced reorientation of DNA strands on organic field-effect transistor-based biosensors sensitivity at high ionic strength. *Applied Physics Letters,* 2015, vol. 107 (10), 103301
- **A. VACCA ; M. MASCIA ; S. RIZZARDINI ; S. PALMAS ; S. LAI ; C. NAPOLI ; M. BARBARO.** Functionalization of Polycrystalline Gold Through the Electroreduction of Aryldiazonium Salts in Ionic Liquids. *Chemical Engineering Transactions,* 2014, vol. 41, 79-84
- **T. M. HEME ; M. J. TARLOV.** Characterization of DNA Probes Immobilized on Gold Surfaces. *Journal of the american chemical society,* 1997, vol. 38 (119), 8916-8920